(19) **Europäisches Patentamt European Patent Office Office européen des brevets**

(11) **EP 4 588 907 A1**

(12) **EUROPEAN PATENT APPLICATION**
published in accordance with Art. 153(4) EPC

(43) Date of publication:
**23.07.2025 Bulletin 2025/30**

(21) Application number: **23865020.4**

(22) Date of filing: **23.06.2023**

(51) International Patent Classification (IPC):
**C07C 17/25** (2006.01)   **B01J 21/04** (2006.01)
**B01J 35/60** (2024.01)   **C07C 21/18** (2006.01)
**C07B 61/00** (2006.01)

(52) Cooperative Patent Classification (CPC):
**B01J 21/04; C07C 17/25; C07C 21/18;** C07B 61/00

(86) International application number:
**PCT/JP2023/023430**

(87) International publication number:
**WO 2024/057657 (21.03.2024 Gazette 2024/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.09.2022 JP 2022148004**
**14.11.2022 JP 2022182072**

(71) Applicant: **AGC INC.**
**Chiyoda-ku,**
**Tokyo 1008405 (JP)**

(72) Inventors:
• **YAMADA, Taku**
**Tokyo 100-8405 (JP)**
• **IWASAKI, Hikaru**
**Tokyo 100-8405 (JP)**
• **OKAMOTO, Hidekazu**
**Tokyo 100-8405 (JP)**
• **OTSUKA, Tetsuo**
**Tokyo 100-8405 (JP)**

(74) Representative: **Müller-Boré & Partner**
**Patentanwälte PartG mbB**
**Friedenheimer Brücke 21**
**80639 München (DE)**

(54) **FLUOROOLEFIN PRODUCTION METHOD**

(57)    The present invention provides a method of producing a fluoroolefin, the method including the step of bringing a fluorocarbon represented by the following Formula (1): $CX^1X^2F\text{-}CX^3X^4H$ (1), into contact with a catalyst to produce a fluoroolefin represented by the following Formula (2): $CX^1X^2=CX^3X^4$ (2), in which in Formula (1) and Formula (2), each of $X^1$, $X^2$, $X^3$ and $X^4$ independently represents a hydrogen atom or a fluorine atom, and at least one of $X^1$, $X^2$, $X^3$ or $X^4$ is a fluorine atom. In the above-described method, the catalyst includes $\alpha$-alumina, and the catalyst has an average pore diameter of 5 nm or more.

EP 4 588 907 A1

**Description**

Technical Field

The present disclosure relates to a method of producing a fluoroolefin.

Background Art

Fluoroolefins are drawing attention in recent years, as compounds having a low global warming potential.
For example, Patent Document 1 discloses a method of producing a fluoroolefin, which method includes a dehydrofluorination step of bringing a fluorocarbon into contact with a metal catalyst to perform the dehydrofluorination of the fluorocarbon, in which the dehydrofluorination step is carried out in a gas phase, in the presence of water, and in which the concentration of water is 500 ppm with respect to the fluorocarbon. In this method, $\gamma$-alumina is used as the catalyst. Further, Non-patent Document 1 discloses a method of obtaining trifluoroethylene, in which method $\alpha$-alumina having an average pore diameter of 3.20 nm is used in the dehydrofluorination reaction of 1,1,1,2-tetrafluoroethane.

Citation List

Patent Document

Patent Document 1: Japanese Patent Application Laid-Open (JP-A) No. 2019-196347 Non-Patent Document
Non-Patent Document 1: Catalysis Letters 2015, Vol. 145, p 654-661

SUMMARY OF INVENTION

Technical Problem

However, conversion rate tends to decrease in a production over a long period of time, in a case in which $\gamma$-alumina is used as the catalyst, as disclosed in Patent Document 1. In a case in which $\alpha$-alumina having an average pore diameter of 3.20 nm is used as the catalyst, as disclosed in Non-patent Document 1, the conversion rate was 1.2%, which is extremely low.
An object of one embodiment of the present disclosure is to provide a method of producing a fluoroolefin, which method is capable of achieving a higher conversion rate as compared to a conventional method, and in which a decrease in the conversion rate is reduced in a production over a long period of time.

Solution to Problem

The present disclosure includes the following embodiments.

<1> A method of producing a fluoroolefin, the method including a step of bringing a fluorocarbon represented by the following Formula (1):

$$CX^1X^2F\text{-}CX^3X^4H \qquad (1)$$

into contact with a catalyst to produce a fluoroolefin represented by the following Formula (2):

$$CX^1X^2{=}CX^3X^4 \qquad (2)$$

(wherein in Formula (1) and Formula (2), each of $X^1$, $X^2$, $X^3$ and $X^4$ independently represents a hydrogen atom or a fluorine atom, and at least one of $X^1$, $X^2$, $X^3$ or $X^4$ is a fluorine atom),
wherein the catalyst contains $\alpha$-alumina, and
wherein the catalyst has an average pore diameter of 5 nm or more.

<2> The method of producing a fluoroolefin according to <1>, wherein the catalyst contains 65% by mass or more of $\alpha$-alumina.
<3> The method of producing a fluoroolefin according to <1> or <2>, wherein the fluorocarbon is brought into contact with the catalyst at a temperature of from 300 to 800°C.

<4> The method of producing a fluoroolefin according to any one of <1> to <3>, wherein a contact time during which between the fluorocarbon is brought into contact with and the catalyst is from 0.5 seconds to 100.0 seconds.

<5> The method of producing a fluoroolefin according to any one of <1> to <4>, wherein a contact time between the fluorocarbon and the catalyst is from 1 g·sec/mL to 200 g·sec/mL.

<6> The method of producing a fluoroolefin according to any one of <1> to <5>, wherein the catalyst has an average pore diameter of from 5 to 200 nm.

<7> The method of producing a fluoroolefin according to any one of <1> to <6>, wherein the catalyst has a pore volume of from 0.002 to 1.20 $cm^3/g$.

<8> The method of producing a fluoroolefin according to any one of <1> to <7>, wherein the catalyst is a molded body.

<9> The method of producing a fluoroolefin according to any one of <1> to <8>, wherein the fluorocarbon is at least one selected from a group consisting of 1,1,1-trifluoroethane, 1,1,2-trifluoroethane, 1,1,2,2-tetrafluoroethane, and 1,1,1,2-tetrafluoroethane.

<10> The method of producing a fluoroolefin according to any one of <1> to <9>, wherein the fluoroolefin is at least one selected from a group consisting of 1,2-difluoroethylene, 1,1-difluoroethylene, and trifluoroethylene.

<11> The method of producing a fluoroolefin according to any one of <1> to <10>, wherein the fluorocarbon is 1,1,1,2-tetrafluoroethane, and the fluoroolefin is trifluoroethylene.

<12> The method of producing a fluoroolefin according to any one of <1> to <11>,

wherein the fluorocarbon is brought into contact with the catalyst in a presence of an inert gas, and

wherein the inert gas is at least one selected from a group consisting of nitrogen, helium, argon, octafluorocyclobutane, and carbon dioxide.

<13> The method of producing a fluoroolefin according to any one of <1> to <12>, further including a step of drying the catalyst, before bringing the fluorocarbon into contact with the catalyst.

<14> The method of producing a fluoroolefin according to any one of <1> to <13>,

wherein the fluorocarbon is brought into contact with the catalyst in a gas phase, in a presence of water, and

wherein a concentration of the water is less than 500 ppm with respect to a total amount of a raw material gas containing the fluorocarbon.

<15> The method of producing a fluoroolefin according to any one of <1> to <14>, wherein a conversion rate 10 hours after bringing the fluorocarbon into contact with the catalyst is 30% or less.

Advantageous Effects of Invention

The present disclosure enables to provide a method of producing a fluoroolefin, which method is capable of achieving a higher conversion rate as compared to a conventional method, and in which a decrease in the conversion rate is reduced in a production over a long period of time.

DESCRIPTION OF EMBODIMENTS

In the present disclosure, a numerical range indicated using the expression "from * to *" represents a range in which numerical values described before and after the preposition "to" are included in the range as a minimum value and a maximum value, respectively.

In numerical ranges described in stages, in the present disclosure, an upper limit value or a lower limit value described in a certain numerical range may be replaced with an upper limit value or a lower limit value of another numerical range in stages. Further, in a numerical range described in the present disclosure, the upper limit value or the lower limit value described in a certain numerical range may be replaced with a value shown in Examples.

In the present disclosure, a combination of two or more preferred embodiments is a more preferred embodiment.

In the present disclosure, the amount of each component refers, in a case in which a plurality of kinds of substances corresponding to each component are present, to the total amount of the plurality of kinds of substances, unless otherwise defined.

[Method of Producing Fluoroolefin]

The method of producing a fluoroolefin according to the present disclosure includes the step of bringing a fluorocarbon represented by the following Formula (1):

$$CX^1X^2F\text{-}CX^3X^4H \qquad (1)$$

into contact with a catalyst to produce a fluoroolefin represented by the following Formula (2):

$$CX^1X^2=CX^3X^4 \qquad (2)$$

(wherein in Formula (1) and Formula (2), each of $X^1$, $X^2$, $X^3$ and $X^4$ independently represents a hydrogen atom or a fluorine atom, and at least one of $X^1$, $X^2$, $X^3$ or $X^4$ is a fluorine atom),

wherein the catalyst contains $\alpha$-alumina, and
wherein the catalyst has an average pore diameter of 5 nm or more.

The method of producing a fluoroolefin according to the present disclosure enables to achieve a higher conversion rate as compared to a conventional method, and to reduce a decrease in the conversion rate in a production over a long period of time. Although the reasons for this are not clear, it is assumed to be as follows.

In the reaction to obtain a fluoroolefin represented by Formula (2) from a fluorocarbon represented by Formula (1), hydrogen fluoride is generated. The generated hydrogen fluoride reacts with the fluoroolefin represented by Formula (2), and the reaction to return to the fluorocarbon represented by Formula (1) proceeds, and therefore, the conversion rate of the fluorocarbon represented by Formula (1) has conventionally been extremely low. To address this problem, the present inventors have found out that the conversion rate is improved when the catalyst contains $\alpha$-alumina, and the catalyst has an average pore diameter of 5 nm or more. The reasons for this are thought to be because the fluorocarbon as a raw material is diffused in the pores of the catalyst, resulting in an increased contact area between the fluorocarbon and the catalyst. In addition, since $\alpha$-alumina has an excellent durability, it is thought that a decrease in the conversion rate is reduced even in a production over a long period of time (at least 50 hours).

In contrast, Patent Document 1 discloses a method of producing a fluoroolefin using $\gamma$-alumina. However, since the crystal structure of $\gamma$-alumina greatly changes in a high temperature environment, the conversion rate tends to decrease in a production over a long period of time in a case in which $\gamma$-alumina is used as the catalyst. Further, Non-patent Document 1 discloses a method of producing trifluoroethylene using $\alpha$-alumina having an average pore diameter of 3.20 nm, and describes that the conversion rate is about 1.2%.

The method of producing a fluoroolefin according to the present disclosure will be described below in detail.

(Fluorocarbon Represented by Formula (1))

In the method of producing a fluoroolefin according to the present disclosure, a fluorocarbon represented by the following Formula (1):

$$CX^1X^2F\text{-}CX^3X^4H \qquad (1)$$

is used as a raw material.

In Formula (1), each of $X^1$, $X^2$, $X^3$ and $X^4$ independently represents a hydrogen atom or a fluorine atom, and at least one of $X^1$, $X^2$, $X^3$ or $X^4$ is a fluorine atom.

Examples of the fluorocarbon represented by Formula (1) include the following compounds:

$CHF_2CH_3$: 1,1-difluoroethane (HFC-152a),
$CH_2FCH_2F$: 1,2-difluoroethane (HFC-152),
$CF_3CH_3$: 1,1,1-trifluoroethane (HFC-143a),
$CHF_2CH_2F$: 1, 1,2-trifluoroethane (HFC-143),
$CF_3CH_2F$: 1,1,1,2-tetrafluoroethane (HFC-134a),
$CHF_2CHF_2$: 1, 1,2,2-tetrafluoroethane (HFC-134), and
$CF_3CHF_2$: 1,1,1,2,2-pentafluoroethane (HFC-125).

Among these compounds, the fluorocarbon represented by Formula (1) is preferably at least one selected from the group consisting of HFC-143a, HFC-143, HFC-134a and HFC-134, from the viewpoint that fewer side reactions are involved, thereby reducing the generation of byproducts. Further, the fluorocarbon represented by Formula (1) is preferably HFC-134a, because a single kind of fluoroolefin can be obtained with a high selectivity.

(Fluoroolefin Represented by Formula (2))

In the method of producing a fluoroolefin according to the present disclosure, a fluoroolefin represented by the following Formula (2):

$$CX^1X^2=CX^3X^4 \qquad (2)$$

is obtained, as a reaction product.

In Formula (2), each of $X^1$, $X^2$, $X^3$ and $X^4$ independently represents a hydrogen atom or a fluorine atom, and at least one of $X^1$, $X^2$, $X^3$ or $X^4$ is a fluorine atom.

Examples of the fluoroolefin represented by Formula (2) include the following compounds:

$CHF=CH_2$: fluoroethylene,
$CF_2=CH_2$: 1,1-difluoroethylene (HFO-1132a),
$CHF=CHF$: 1,2-difluoroethylene (HFO-1132(E), HFO-1132(Z)),
$CHF=CF_2$: trifluoroethylene (HFO-1123), and
$CF_2=CF_2$: tetrafluoroethylene.

Among these compounds, the fluoroolefin represented by Formula (2) is preferably at least one selected from the group consisting of HFO-1132, HFO-1132a and HFO-1123, from the viewpoint of usability as a coolant composition.

In the method of producing a fluoroolefin according to the present disclosure, in particular, the fluorocarbon is preferably HFC-134a and the fluoroolefin is preferably HFO-1123, from the viewpoint of that the reaction proceeds more selectively.

(Catalyst)

In the method of producing a fluoroolefin according to the present disclosure, a fluorocarbon represented by Formula (1) is brought into contact with a catalyst.

The catalyst to be brought into contact with the fluorocarbon contains $\alpha$-alumina, and the catalyst has an average pore diameter of 5 nm or more.

Alumina is a dehydration product of aluminum hydroxide, and has different properties depending on the degree of dehydration and the degree of crystallinity. There are various types of alumina, such as $\gamma$-alumina, $\theta$-alumina and $\alpha$-alumina, depending on the crystal structures thereof. $\gamma$-alumina and $\theta$-alumina are each referred to as an activated alumina, have a higher free energy of formation than that of $\alpha$-alumina, and are thermodynamically unstable. $\alpha$-alumina is a high-temperature stable phase of alumina which has a high degree of crystallinity, and considered to be thermally stable and to have a high thermal conductivity although having a small specific surface area. The catalyst to be used in the method of producing a fluoroolefin according to the present disclosure contains $\alpha$-alumina. $\alpha$-alumina has a higher conversion barrier from Al-O to Al-F in the presence of hydrogen fluoride, as compared to other alumina structures, and the use of a catalyst containing $\alpha$-alumina makes it possible to reduce the generation of $AlF_3$. The generation of $AlF_3$ is thought to lead to the inactivation of the catalyst, a decrease in the selectivity and the like.

When the catalyst contains $\alpha$-alumina, the catalyst has a higher durability, and a decrease in the conversion rate is reduced even in a production over a long period of time. Further, when the catalyst has an average pore diameter of 5 nm or more, the fluorocarbon is diffused in the pores of the catalyst, and the contact area between the fluorocarbon and the catalyst is increased, thereby improving the conversion rate.

The fact that the catalyst contains $\alpha$-alumina can be confirmed by X-ray diffractometry, in other words, by a diffraction pattern obtained by an XRD (X-Ray diffractometer). A commercially available apparatus can be used as the XRD. For example, it is possible to use "SmartLab" manufactured by Rigaku Corporation. It can be determined that the catalyst contains $\alpha$-alumina, in a case in which peaks at d = 26.62, 35.21, 37.85, 43.43, 52.65 and 57.61 Å are present in the diffraction pattern. The above-described analysis is carried out for a catalyst which is immediately before being brought into contact with the fluorocarbon, or a catalyst in which the same state as the catalyst immediately before being brought into contact with the fluorocarbon is reproduced.

The catalyst may contain a compound other than $\alpha$-alumina. The compound other than $\alpha$-alumina may be, for example, alumina having a structure different from the crystal structure of $\alpha$-alumina, an oxide other than alumina, or the like. Examples of the alumina having a structure different from the crystal structure of $\alpha$-alumina include $\beta$-alumina, $\gamma$-alumina, $\theta$-alumina, $\eta$-alumina, boehmite, and gibbsite. Examples of the oxide other than alumina include chromium oxide, copper oxide, iron oxide, nickel oxide, magnesium oxide, zinc oxide, and zirconium oxide.

Further, the catalyst may contain, for example, fluorinated aluminum oxide obtained by fluorinating $\alpha$-alumina, or aluminum fluoride, as the compound other than $\alpha$-alumina.

**EP 4 588 907 A1**

The catalyst to be brought into contact with the fluorocarbon preferably contains $\alpha$-alumina as a main component. Specifically, the catalyst preferably contains 65% by mass or more of $\alpha$-alumina when the mass of the catalyst is taken as 100% by mass. When the catalyst contains $\alpha$-alumina as a main component, the catalyst has a higher durability, and a decrease in the conversion rate is reduced even in a production over a long period of time.

The fact that the catalyst contains 65% by mass or more of $\alpha$-alumina can be confirmed by performing a Rietveld analysis based on the crystal structure determined by the XRD. Specifically, the mass proportion of each crystal structure is calculated, by comparing the peaks obtained by the XRD measurement of the catalyst with known peak models derived from the respective alumina structures, and performing the Rietveld analysis of the peaks. The catalyst preferably contains 65% by mass or more, more preferably 70% by mass or more, still more preferably 75% by mass or more, particularly preferably 80% by mass or more, and most preferably 85% by mass or more, of $\alpha$-alumina. The catalyst may contain 100% by mass of $\alpha$-alumina, namely, the catalyst may consist of $\alpha$-alumina.

In the identification of the crystal structures by the XRD, the peaks at d = 26.62, 35.21, 37.85, 43.43, 52.65 and 57.61 Å are used as the peaks derived from $\alpha$-alumina, as described above.

$\alpha$-alumina may not only function as a catalyst, but also function as a carrier while functioning as a catalyst. Further, $\alpha$-alumina may be supported on a carrier other than $\alpha$-alumina.

Examples of the carrier include carbon, $\beta$-alumina, $\gamma$-alumina, zirconia, silica, and titania.

The form of the catalyst is not particularly limited, and the catalyst may be in the form of a powder, pellets, or spheres.

In the case of using $\alpha$-alumina for around 10 hours, the $\alpha$-alumina is preferably a molded body, such as one in the form of pellets or spheres, from the viewpoint of handleability, because excellent filling properties at the time of filling the $\alpha$-alumina into a reactor and an excellent flowability of a reaction gas can be achieved.

Unlike powder, the molded body is, for example, one obtained by introducing a powder into a mold and subjecting the powder to compression molding.

So far, a plurality of methods have been proposed for determining the pore structures of catalysts. One of the most commonly used methods is a method of measuring an adsorption isotherm by a gas adsorption method. Nitrogen can be used as a common adsorption gas for analyzing pores in microporous (less than 2 nm) and mesoporous (from 2 to 200 nm) ranges. Parameters such as specific surface area, average pore diameter and pore volume can be obtained, from adsorption and desorption isotherms.

- Specific Surface Area -

In general, it is understood that the higher the surface area of a substrate which can be brought into contact with a catalyst, the more improved the contact frequency between the substrate and the catalyst surface, and thus, the higher the catalytic activity. Decreasing the average pore diameter and the pore volume of a catalyst is known to increase the specific surface area of the catalyst. Therefore, there are extremely few cases in which $\alpha$-alumina, which has the lowest specific surface area among alumina having a plurality of crystal structures, is used as a catalyst. However, contrary to the understanding so far, the inventors have discovered that increasing in the average pore diameter that causes a decrease in the specific surface area, in $\alpha$-alumina, leads to a higher activity of the reaction in the present disclosure. The specific surface area of the catalyst can be calculated from the monolayer adsorption of nitrogen, based on the theory of BET (Brunauer-Emmett-Teller).

- Average Pore Diameter -

The average pore diameter can be calculated based on the theory of BJH (Barrett-Joyner-Halenda).

The average pore diameter of the catalyst is measured by the following method.

In a case in which the catalyst has an average pore diameter of from 2 to 200 nm, the average pore diameter can be determined by the BET method and the BJH method based on the nitrogen adsorption method. For example, "3Flex" manufactured by Micromeritics Instrument Corp. can be used as a measuring apparatus.

The average pore diameter is calculated using the specific surface area (S) determined by the BET method from the adsorption isotherm obtained by the nitrogen adsorption method, and the pore volume (V) determined by the BJH method, based on the following Equation.

$$D = 4V/S$$

In the above Equation, D represents the average pore diameter (m) of a sample, V represents the pore volume ($m^3/g$) of the sample, and S represents the specific surface area ($m^2/g$) of the sample.

In a case in which the catalyst has an average pore diameter of less than 2 nm, on the other hand, the average pore diameter is calculated by the HK (Horvath-Kawazoe) method in which a Lennard-Jones function is used.

This measurement is carried out immediately before bringing the catalyst into contact with the fluorocarbon.

When the catalyst has a larger average pore diameter, it means that there are pore spaces having a larger diameter on the surface and in the interior of the catalyst. Therefore, the diffusivity of the fluorocarbon is improved, and the contact frequency between the catalyst surface and the fluorocarbon is increased, making it possible to expect an improvement in the reactivity. Further, too large an average pore diameter tends to result in a decrease in the specific surface area of the catalyst, and to reduce an effect as a catalyst.

From the viewpoints described above, the catalyst has an average pore diameter of 5 nm or more, and preferably from 5 to 200 nm.

The average pore diameter of the catalyst is preferably 6 nm or more, more preferably 10 nm or more, still more preferably 12 nm or more, and particularly preferably 15 nm or more. Further, the average pore diameter of the catalyst is more preferably 150 nm or less, still more preferably 100 nm or less, particularly preferably 50 nm or less, and most preferably 30 nm or less, from the viewpoint of ensuring the specific surface area.

- Pore Volume -

The pore volume can be calculated based on the theory of BJH (Barrett-Joyner-Halenda).

The pore volume has an impact on the diffusivity of a substrate into the interior of the pores of the catalyst, and on the strength of the catalyst. When the catalyst has a larger pore volume, the diffusivity of the fluorocarbon into the interior of the pores of the catalyst is improved, and the contact frequency between the catalyst and the fluoroolefin is increased. As a result, an improvement in the reactivity can be expected. Further, too large a pore volume tends to result in a decrease in the strength of the catalyst, and in a decrease in the strength of the catalyst.

From the viewpoints described above, the catalyst preferably has a pore volume of from 0.002 to 1.20 $cm^3/g$.

The pore volume of the catalyst is more preferably 0.005 $cm^3/g$ or more, still more preferably 0.01 $cm^3/g$ or more, and particularly preferably 0.02 $cm^3/g$ or more. The pore volume of the catalyst is more preferably 1 $cm^3/g$ or less, still more preferably 0.90 $cm^3/g$ or less, and particularly preferably 0.80 $cm^3/g$ or less.

- Bulk Density-

The bulk density of the catalyst is not particularly limited. However, when the catalyst has a high bulk density, the catalyst has a smaller volume per the same mass, making it possible to reduce the size of the reactor to be used, which is efficient. When the catalyst has a low bulk density, on the other hand, the specific surface area and the pore volume of the catalyst are increased.

From the viewpoints described above, the bulk density of the catalyst is preferably from 0.4 to 1.5 g/mL.

The bulk density of the catalyst is more preferably 0.5 g/mL or more, still more preferably 0.6 g/mL or more, and particularly preferably 0.7 g/mL or more. Further, the bulk density of the catalyst is more preferably 1.4 g/mL or less, still more preferably 1.3 g/mL, and particularly preferably 1.2 g/mL or less.

(Reaction Conditions)

In the method of producing a fluoroolefin according to the present disclosure, the raw material gas is required to contain a fluorocarbon represented by Formula (1), and may contain a component other than the fluorocarbon represented by Formula (1). The raw material gas may consist of a fluorocarbon represented by Formula (1), or may contain any of isomers obtained during the production of the fluorocarbon represented by Formula (1), disproportionation products, impurities and the like. From the viewpoint of reducing side reactions and catalyst inactivation, the raw material gas preferably contains an inert gas such as nitrogen, argon, helium, carbon dioxide or octafluorocyclobutane, in addition to the fluorocarbon represented by Formula (1). The inert gas is capable of diluting a target product and hydrogen fluoride as a byproduct. The content of the fluorocarbon represented by Formula (1) is preferably 60% by mole or more, more preferably 70% by mole or more, still more preferably 75% or more, and particularly preferably 80% or more, with respect to the total amount of the raw material gas.

The method of producing a fluoroolefin according to the present disclosure may be carried out in a gas phase, or in a liquid phase. Since the fluorocarbon represented by Formula (1) is a gas at normal temperature, the fluorocarbon is preferably brought into contact with the catalyst in the gas phase.

The reactor in which the fluorocarbon is brought into contact with the catalyst can be any reactor capable of withstanding the temperature and pressure to be described later, and the shape and the structure of the reactor are not particularly limited. The reactor may be, for example, a cylindrical vertical reactor. The reactor may be made of a material, such as, for example, glass, stainless steel, iron, nickel, an alloy containing iron or nickel as a main component, or the like. The reactor may include a heating means such as an electric heater for heating the interior of the reactor.

The catalyst may be accommodated in a reactor with any of a fixed bed, a fluidized bed or a moving bed form. In the case of using a fixed bed reactor, either a horizontal fixed bed reactor or a vertical fixed bed reactor may be used.

Further, the reaction may be carried out either in a flow mode or a batch mode.

In a fixed bed reactor, any of various types of molded bodies of a catalyst-supporting carrier is filled into the reactor, in order to reduce the pressure loss of a reaction fluid. Further, a type of reactor in which the catalyst is filled thereinto in the same manner as the fixed bed reactor, transferred by the gravity thereof, and extracted from the bottom of the reactor to be regenerated, for example, is referred to as a "moving bed reactor". In a fluidized bed reactor, catalyst particles are suspended in a reaction fluid and transferred inside the reactor, because an operation is carried out in which a catalyst layer shows properties as if the layer is a fluid, due to the reaction fluid. A fixed bed reactor is preferred, from the viewpoints that it allows for a wide selection of the shape of the catalyst, and that the wear of the catalyst can be reduced. There are a tubular reactor and a tank reactor as the types of the fixed bed reactor, and a tubular reactor is preferred from the viewpoint of the ease of control of reaction temperature. Further, it is possible to use, for example, a multi-tube heat exchange reaction in which a large number of reaction tubes having a small tube diameter are arranged in parallel, and a heating medium is circulated externally. In a case in which a plurality of reactors are provided in series, a plurality of catalyst layers are provided. It is required that at least one catalyst layer is provided, and two or more catalyst layers may be provided.

The method of producing a fluoroolefin according to the present disclosure is preferably carried out in a flow mode using a fixed bed reactor (particularly, a vertical fixed bed reactor), from the viewpoint of improving the conversion rate.

In the method of producing a fluoroolefin according to the present disclosure, it is preferred that the fluorocarbon is brought into contact with catalyst at a temperature of from 300 to 800°C, more preferably at a temperature of from 400 to 700°C, and still more preferably at a temperature of from 400 to 600°C. When the fluorocarbon is brought into contact with catalyst at a contact temperature of 300°C or higher, the conversion rate of the fluoroolefin is improved. When the contact temperature is 800°C or lower, on the other hand, the decomposition of the fluoroolefin can be reduced.

When the contact temperature is 300°C or higher, the reaction proceeds adequately. When the contact temperature is 800°C or lower, on the other hand, a decrease in selectivity due to carbon-carbon bond cleavage in the raw material, and the disproportionation reaction of the resulting product (unsaturated compound) are reduced.

Since the dehydrofluorination reaction is an endothermic reaction, in general, a decrease in the conversion rate can be reduced by adequately maintaining the reaction temperature. An increase in the reaction temperature in the catalyst layer leads to an increase in the conversion rate of the raw material. Therefore, it is preferred to maintain the reaction temperature in the catalyst layer at a desired temperature so that a high conversion rate can be maintained. To maintain the reaction temperature in the catalyst layer at a desired temperature, it is possible to use, for example, a method in which the catalyst layer is externally heated by a heating medium or the like. The catalyst usually degrades over time as the reaction proceeds. Even in a case in which a decrease in the conversion rate of the raw material due to the degradation of the catalyst has occurred, it is possible to reduce a decrease in the conversion rate by heating the catalyst layer with a heating medium or the like, to adequately maintain or increase the reaction temperature. At the time of maintaining or increasing the temperature of the catalyst layer, it is preferred to adjust the range of temperature increase within 50°C or less in order to reduce a rapid degradation of the catalyst.

At the start of the reaction, the reaction zone starts from an introduction portion through which the raw material gas is introduced. When the catalyst at the introduction portion of the raw material gas degrades over time as the reaction proceeds, the reaction zone shifts downstream in the flow direction of the gas. Since a low-temperature gas generated in the reaction zone flows into the vicinity of the downstream side of the reaction zone, the vicinity of the downstream side usually has the lowest temperature in the catalyst layer. In the present disclosure, the above-described temperature of the region of the catalyst layer having the lowest temperature is referred to as "the lowest temperature of the catalyst layer". The temperature of the region further downstream of the vicinity of the downstream side usually increases from the lowest temperature of the catalyst layer, as it gets farther away from the reaction zone.

In the method of producing a fluoroolefin according to the present disclosure, the raw material gas containing the fluorocarbon may be supplied to a reactor as it is at normal temperature. However, it is preferred to heat (preheat) the raw material gas adequately before supplying the gas to the reactor, due to the reason to be described later. In the case of performing preheating, the raw material gas is preferably supplied to the reactor after being heated to a temperature of from 80 to 600°C. When the raw material gas is preheated to 80°C or higher, the internal temperature of the reactor is not easily decreased, making it easier to achieve the conversion rate that has been set. When the raw material gas is preheated to 600°C or lower, the internal temperature of the reactor is not easily increased, undesired reactions are reduced, and the selectivity is improved.

Since the dehydrofluorination reaction in the present disclosure is a reaction in which the number of molecules increases, increasing the pressure is disadvantageous for a forward reaction.

The pressure when the fluorocarbon is brought into contact with the catalyst is not particularly limited. However, the pressure is preferably from -0.05 to 2MPa, more preferably from -0.01 to 1MPa, and still more preferably from normal pressure to 0.5MPa, from the viewpoint of improving the conversion rate.

In the present disclosure, the "pressure" refers to "gauge pressure".

The contact time between the fluorocarbon and the catalyst is from 0.5 seconds to 100.0 seconds, and more preferably from 1.0 to 50.0 seconds. The contact time is still more preferably from 2.0 to 20.0 seconds.

The contact time (seconds) described above is calculated using the following Equation.

Contact time (seconds) = [length (cm) to which catalyst is filled in reactor]/[linear velocity (cm/seconds)]

The "linear velocity" refers to a velocity at which the fluorocarbon passes through the catalyst per unit of time.

Further, the contact time (g·sec/mL) during which the fluorocarbon is brought into contact with the catalyst is preferably from 1 to 200 g·sec/mL, more preferably from 5 to 175 g·sec/mL, still more preferably from 7 to 150 g·sec/mL, and particularly preferably from 10 to 125 g·sec/mL. When the contact time (g·sec/mL) is 1 g·sec/mL or more, the conversion rate is improved. When the contact time (g·sec/mL) is 200 g·sec/mL or less, the cost of equipment can be reduced.

The contact time (g·sec/mL) described above is calculated using the following Equation.

Contact time (g·sec/mL) = [filling amount (g) of catalyst]/[flow rate (mL/seconds) of fluorocarbon]

It is preferred that the fluorocarbon is brought into contact with the catalyst in the presence of an inert gas, from the viewpoint of further reducing a decrease in the conversion rate. The inert gas is preferably at least one selected from the group consisting of nitrogen, helium, argon, octafluorocyclobutane, and carbon dioxide. Among these gases, the inert gas is preferably nitrogen.

The molar ratio of the fluorocarbon with respect to the inert gas, in the gas phase, is preferably from 0.1 to 30, and more preferably from 0.5 to 25.

From the viewpoint of further reducing a decrease in the conversion rate, it is preferred that the fluorocarbon is brought into contact with the catalyst in the gas phase, in the presence of water, and that the concentration of the water is less than 500 ppm with respect to the total amount of a raw material gas containing the fluorocarbon.

It is thought that the dehydrofluorination reaction in the present disclosure proceeds due to Lewis acid sites on the surface of the catalyst serving as active sites. By performing the dehydrofluorination reaction in the gas phase, in the presence of water, the water is adsorbed on the Lewis acid sites on the surface of the catalyst. When the concentration of the water is adjusted to less than 500 ppm with respect to the total amount of the raw material gas containing the fluorocarbon, it is assumed that the Lewis acid sites on the surface of the catalyst are destroyed to form structures similar to Brønsted acid sites, thereby reducing a decrease in the activity of the catalyst.

A common method of measuring the concentration of water may be, for example, a method of using a commercially available Karl Fischer moisture meter. When the concentration of the water is less than 500 ppm with respect to the total amount of the fluorocarbon, the conversion rate is increased, and the target product can be obtained with a high selectivity. The concentration of the water is preferably 300 ppm or less, more preferably 100 ppm or less, still more preferably 50 ppm or less, and particularly preferably 10 ppm or less, because the conversion rate can be improved even further, and the target compound can be obtained with an even higher selectivity. Although the concentration of the water is that the lower is more preferred, the concentration of the water is preferably 0.5 ppm or more, and more preferably 1 ppm or more, from the viewpoint of the cost of dehydration treatment of the fluorocarbon and the inert gas, and the viewpoint of preventing process management from becoming complicated.

The concentration of the water described above is the content of the water contained in the raw material gas, when the fluorocarbon is brought into contact with the catalyst. The expression "the concentration of the water" may be replaced with the expression "the content of the water contained in the raw material gas before allowing the gas to flow into a reactor".

The method of producing a fluoroolefin according to the present disclosure preferably further includes the step of drying the catalyst, before bringing the fluorocarbon into contact with the catalyst. By drying the catalyst, the water contained in the catalyst is removed, and the reactivity to the fluorocarbon is increased, thereby improving the conversion rate.

The method of drying the catalyst is not particularly limited. The catalyst may be dried before filling the catalyst into a reactor, or after filling the catalyst into the reactor. In the case of drying the catalyst after filling the catalyst into a reactor, the reactor can be preheated while drying the catalyst, and thus is preferred. Specifically, the catalyst is preferably dried by filling the catalyst into a reactor, and heating the reactor while allowing an inert gas to flow.

In the method of producing a fluoroolefin according to the present disclosure, hydrogen fluoride is generated as a side product. Hydrogen fluoride has a function of fluorinating the oxide(s) contained in the catalyst, to enhance acidity. Therefore, it is preferred to decrease the concentration of hydrogen fluoride. When the concentration of hydrogen fluoride is decreased, the selectivity is maintained, the inactivation of the catalyst is reduced, and a decrease in reaction activity due to a decrease in the specific surface area can be reduced.

The concentration of hydrogen fluoride can be decreased, for example, by a method of diluting the hydrogen fluoride with an inert gas.

Since the use of an inert gas leads to an increase in energy load of purification process after the reaction, it is preferred to adequately control the use of the inert gas.

The concentration of hydrogen fluoride during the reaction is preferably adjusted to 15% by mole or less. The concentration of hydrogen fluoride is more preferably 13% by mole or less, sill more preferably 100% by mole or less, particularly preferably 8% by mole or less, and most preferably 7% by mole or less, from the viewpoint of prolonging catalyst life. The concentration of hydrogen fluoride is preferably 0.5% by mole or more, more preferably 0.8% by mole or more, still more preferably 1.0% by mole or more, particularly preferably 1.3% by mole or more, and most preferably 1.5% by mole or more, from the viewpoints of productivity and the energy load of the purification process.

The method of producing a fluoroolefin according to the present disclosure is preferably performed in the presence of an oxidizing agent. The oxidizing agent is preferably oxygen, chlorine, bromine or iodine, because the conversion rate can be increased, and the target compound can be obtained with a high selectivity. Among these oxidizing agents, oxygen is more preferred. In the present disclosure, the concentration of the oxidizing agent is preferably from 0.01 to 21% by mole with respect to the raw material gas. The concentration of the oxidizing agent is more preferably from 1 to 20% by mole, still more preferably from 5 to 18% by mole, and particularly preferably from 7.5 to 16% by mole, with respect to the raw material compound, because the conversion rate can further be improved, and the target compound can be obtained with a higher selectivity.

In the present disclosure, the "conversion rate" refers to the proportion (% by mole) of the total molar amount of compounds other than the raw material compound contained in the gas flowed out from a reactor outlet, with respect to the molar amount of the raw material compound supplied to the reactor.

Specifically, the conversion rate is a conversion rate 10 hours after bringing the fluorocarbon into contact with the catalyst.

In general, a higher conversion rate is preferred from the viewpoint of the productivity. However, it is preferred to adequately control the conversion rate, in the dehydrofluorination reaction in the present disclosure. When the conversion rate is controlled, it is thought that the concentration of hydrogen fluoride generated in the gas phase is decreased, and the inactivation of the catalyst by hydrogen fluoride is reduced. Using a method of diluting the gas phase with an inert gas is also plausible. However, since the use of an inert gas causes an increase in the energy load of the purification process after the reaction, an excessive use of the inert gas is not preferred. From the viewpoint of prolonging the catalyst life, the concentration of hydrogen fluoride during the reaction is preferably reduced to 15% by mole or less. Accordingly, in a case in which the amount of the inert gas used is equal to or less than the equivalent of the fluoroolefin, it is preferred to select operation conditions in which the conversion rate is set to 30% or less. The conversion rate is preferably 25% or less, more preferably 20% or less, still more preferably 15% or less, and particularly preferably 13% or less. Too low a conversion rate leads to a decrease in the productivity and to an increase in the size of equipment, and therefore, it is preferred to select operation conditions in which the conversion rate is set to 1.5% or more. The conversion rate is preferably 2% or more, more preferably 2.5% or more, still more preferably 3% or more, and particularly preferably 3.5% or more.

In the method of producing a fluoroolefin according to the present disclosure, in particular, the conversion rate can be prevented from being too high, and the inactivation of the catalyst is reduced, by using HFC-134a as the fluorocarbon.

In the present disclosure, the "selectivity" refers to the proportion (% by mole) of the molar amount of the target product contained in the reactor outlet gas, with respect to the total molar amount of compounds other than the raw material compound contained in the reactor outlet gas.

Specifically, the selectivity is a selectivity 10 hours after bringing the fluorocarbon into contact with the catalyst.

The selectivity is preferably 100%, because no purification process after the reaction will be required. However, side reactions can occur in the range of reaction temperature required for obtaining a desired conversion rate. A higher selectivity is more preferred, because it makes it possible to decrease the amount of waste, to decrease the energy load of the purification process after the reaction, and to prolong the catalyst life. The selectivity is preferably 90% or more, more preferably 93% or more, and still more preferably 95% or more.

In the method of producing a fluoroolefin according to the present disclosure, in particular, HFO-1123 can be obtained with a high selectivity, by using HFC-134a as the fluorocarbon.

Examples of the compounds other than the raw material compound and the target product contained in the reactor outlet gas include HFC-134, 1,1-difluoroethylene (VdF), E/Z-1,2-difluoroethylene (E/Z-HFO-1132), hydrogen fluoride, carbon monoxide, carbon dioxide, and water.

The method of producing a fluoroolefin according to the present disclosure enables to reduce a decrease in the conversion rate in a production over a long period of time (specifically, 50 hours or more). The conversion rate after 10 hours with respect to the conversion rate after 50 hours is preferably 70% or more, more preferably 75% or more, and still more preferably 80% or more.

EXAMPLES

The present disclosure will be described below further specifically with reference to Examples. However, the present disclosure is not limited to the following Examples, as long as it does not depart from the gist of the present disclosure.

The content of $\alpha$-alumina contained in the catalyst used in each of Examples 1 to 13 was measured.

(Content of $\alpha$-alumina in Catalyst)

The content of $\alpha$-alumina in the catalyst was measured, by carrying out an XRD measurement using an X-RAY diffractometer "SmartLab" manufactured by Rigaku Corporation, and performing the Rietveld analysis of the diffraction peaks of the respective crystal phases. The content of $\alpha$-alumina was evaluated in accordance with the following evaluation criteria.

A: The content of $\alpha$-alumina is 65% by mass or more.
B: The content of $\alpha$-alumina is less than 65% by mass.

[Example 1]

$\alpha$-alumina (product name: "$\alpha$-Alumina, average particle diameter: 0.5 $\mu$m", manufactured by FUJIFILM Wako Pure Chemical Corporation) was analyzed by X-Ray diffractometry. As a result, the evaluation result of the content of $\alpha$-alumina was A. One mL of the above-described catalyst was weighed, and used as the catalyst. The catalyst was filled into a reaction tube made of stainless steel (SUS304) and having an inner diameter of 1.02 cm and a length of 30 cm, and the reaction tube was placed in a tubular electric furnace. The portion of the reaction tube filled with the catalyst was heated to 475°C in the tubular electric furnace under a flow of nitrogen, to dehydrate the catalyst. Thereafter, a mixed gas of nitrogen/HFC-134a at a ratio of 0.1/1 (mol/mol) was allowed to flow for a contact time of 4.7 seconds, to carry out a de-HF reaction to produce HFO-1123.

The concentration of the water in the mixed gas of nitrogen/HFC-134a at a ratio of 0.1/1 (mol/mol), as measured with a Karl Fischer moisture meter, was 5 ppm.

[Example 2 to Example 12]

In each of Example 2 to Example 12, the de-HF reaction was carried out in the same manner as in Example 1, except for changing the catalyst, and changing the respective conditions to the values shown in Table 1 and Table 2.

The catalysts used in Example 2 to Example 12 will be described below.

(Example 2)

$\gamma$-alumina (product name: "N612N", manufactured by JGC C&C) was crushed in a mortar to obtain a powder. The resulting powder was calcined at 1,300°C for 6 hours in an air atmosphere, and then analyzed by X-ray diffractometry. As a result, the evaluation result of the content of $\alpha$-alumina was A. The thus obtained powder was used as the catalyst.

(Example 3)

$\gamma$-alumina (product name: "N612N", manufactured by JGC C&C) was calcined at 1,300°C for 6 hours in an air atmosphere. The resulting pellets were analyzed by X-ray diffractometry. As a result, the evaluation result of the content of $\alpha$-alumina was A. The thus obtained pellets were used as the catalyst.

(Example 4)

$\alpha$-alumina (product name: "SA52124", manufactured by Saint-Gobain Ltd.) was used as the catalyst.

(Example 5)

$\alpha$-alumina (product name: "SA52238", manufactured by Saint-Gobain Ltd.) was used as the catalyst.

(Example 6)

α-alumina (product name: "FGL-30", manufactured by Iwatani Chemical Industry Co., Ltd.) was used as the catalyst.

(Example 7)

α-alumina (product name: "FGL-40", manufactured by Iwatani Chemical Industry Co., Ltd.) was used as the catalyst.

(Example 8)

α-alumina (product name: "C500", manufactured by Nippon Light Metal Company, Ltd.) was used as the catalyst.

(Example 9)

α-alumina (product name: "LT303D", manufactured by Nippon Light Metal Company, Ltd.) was used as the catalyst.

(Example 10)

α-alumina (product name: "AKQ-10", manufactured by Sumitomo Chemical Co., Ltd.) was used as the catalyst.

(Example 11)

γ-alumina (product name: "N612N", manufactured by JGC C&C) was used as the catalyst.

(Example 12)

α-alumina (product name: "FGH-10", manufactured by Iwatani Chemical Industry Co., Ltd.) was used as the catalyst.

[Example 13]

In Example 13, the de-HF reaction was carried out in the same manner as in Example 8, except that HFC-143 was used instead of HFC-134a.
The average pore diameter of each catalyst was calculated using the specific surface area (S) determined by the BET method from the adsorption isotherm obtained by the nitrogen adsorption method, using "3Flex" manufactured by Micromeritics Instrument Corp., and the pore volume (V) determined by the BJH method, based on the following Equation.

$$D = 4V/S$$

In the above Equation, D represents the average pore diameter (m) of a sample, V represents the pore volume ($m^3$/g) of the sample, and S represents the specific surface area ($m^2$/g) of the sample.
In Example 11 in which it was unable to measure the average pore diameter by the method described above, the average pore diameter was calculated by the HK method in which a Lennard-Jones function is used.
The contact time (seconds) was calculated using the following Equation.

Contact time (seconds) = [length (cm) to which catalyst is filled in reactor]/[linear velocity (cm/seconds)]

The "linear velocity" refers to a velocity at which the fluorocarbon passes through the catalyst per unit of time.
The contact time (g·sec/mL) was calculated using the following Equation.

Contact time (g·sec/mL) = [filling amount (g) of catalyst]/[flow rate (mL/seconds) of fluorocarbon]

In the reaction of each of Example 1 to Example 12, the generated gas (hereinafter, also referred to as "reactor outlet gas") recovered from the outlet of the reactor was analyzed with a gas chromatograph, 10 hours after the start of the reaction. Specifically, a column (product name: "DB-1301", manufactured by Agilent Technologies Inc.; length: 60 m, inner diameter: 0.25 mm, film thickness: 1 $\mu$m) was attached to a gas chromatograph (product name: "GC 6850", manufactured by Agilent Technologies Inc.), and used for the analysis. Using the molar amount calculated from the area ratio (GC Area %) of the reactor outlet gas, the conversion rate of HFC-134a and the selectivity of HFO-1123 were calculated.

(Conversion Rate of HFC-134a)

The conversion rate of HFC-134a refers to the proportion (% by mole) of the total molar amount M1 of components other than HFC-134a contained in the reactor outlet gas, with respect to the molar amount $M_{134a}$ of the HFC-134a supplied to the reactor.

$$\text{Conversion rate (\%) of HFC-134a} = (M1/M_{134a}) \times 100$$

(Selectivity of HFO-1123)

The selectivity of HFO-1123 refers to the proportion (% by mole) of the molar amount $M_{1123}$ of the HFO-1123 contained in the reactor outlet gas, with respect to the total molar amount M1 of compounds other than HFC-134a contained in the reactor outlet gas.

$$\text{Selectivity (\%) of HFO-1123} = (M_{1123}/M1) \times 100$$

In Example 1 and Example 11, the conversion rate of HFC-134a was also calculated 50 hours after the start of the reaction, in the same manner as calculating the conversion rate 10 hours after the start of the reaction. In Examples 2 to 10 and 12, the measurement 50 hours after the start of the reaction was not performed, and thus, the corresponding cells in Table 1 and Table 2 are indicated with "-". In Example 2 to Example 7, as well, it is assumed that a decrease in the conversion rate of HFC-134a 50 hours after the start of the reaction is reduced, in the same manner as in Example 1.
In the reaction of Example 13, the conversion rate of HFC-143 as well as the total selectivity of HFO-1132(E) and HFO-1132(Z) were calculated in the same manner as in Example 1, 10 hours and 50 hours after the start of the reaction.

(Conversion Rate of HFC-143)

The conversion rate of HFC-143 refers to the proportion (% by mole) of the total molar amount M2 of components other than HFC-143 contained in the reactor outlet gas, with respect to the molar amount $M_{143}$ of the HFC-143 supplied to the reactor.

$$\text{Conversion rate (\%) of HFC-143} = (M2/M_{143}) \times 100$$

(Total Selectivity of HFO-1132(E) and HFO-1132(Z))

The total selectivity of HFO-1132(E) and HFO-1132(Z) refers to the proportion (% by mole) of the total molar amount $M_{1132}$ of the HFO-1132(E) and HFO-1132(Z) contained in the reactor outlet gas, with respect to the total molar amount M2 of compounds other than HFC-143 contained in the reactor outlet gas.

$$\text{Total selectivity (\%) of HFO-1132(E) and HFO-1132(Z)} = (M_{1132}/M2) \times 100$$

[Table 1]

| | | | Example 1 | Example 2 | Example 3 | Example 4 | Example 5 | Example 6 |
|---|---|---|---|---|---|---|---|---|
| Fluorocarbon | | | HF-C-134a | HF-C-134a | HF-C-134a | HF-C-134a | HF-C-134a | HFC-134-a |
| Catalyst | Type | | $\alpha$-Al$_2$O$_3$ | $\alpha$-Al$_2$O$_3$ | $\alpha$-Al$_2$O$_3$ | $\alpha$-Al$_2$O$_3$ | $\alpha$-Al$_2$O$_3$ | $\alpha$-Al$_2$O$_3$ |
| | Evaluation result of content of $\alpha$-alumina | | A | A | A | A | A | A |
| | Specific surface area [m$^2$/g] | | 4 | 2 | 3 | 4 | 7 | 2 |
| | Pore volume [cm$^3$/g] | | 0.01 | 0.01 | 0.01 | 0.03 | 0.02 | 0.01 |
| | Average pore diameter [nm] | | 13 | 26 | 20 | 25 | 13 | 9 |
| Contact temperature [°C] | | | 450 | 475 | 475 | 450 | 450 | 450 |
| Pressure [MPaG] | | | 0 | 0 | 0 | 0 | 0 | 0 |
| Molar ratio of Fluorocarbon /N$_2$ [mol/mol] | | | 10 | 10 | 10 | 10 | 10 | 10 |
| Contact time [seconds] | | | 4.7 | 4.6 | 4.6 | 4.7 | 4.7 | 4.7 |
| Contact time [g·sec/mL] | | | 14 | 14 | 27 | 15 | 11 | 17 |
| Conversion Rate [%] | After 10 h | | 10 | 10 | 7.5 | 11.8 | 12.1 | 8.1 |
| | After 50 h | | 8.1 | - | - | - | - | - |
| Selectivity [%] | | | 99 | 99 | 98 | 99 | 99 | 100 |

[Table 2]

| | | Example 7 | Example 8 | Example 9 | Example 10 | Example 11 | Example 12 | Example 13 |
|---|---|---|---|---|---|---|---|---|
| Fluorocarbon | | HF-C-134a | HF-C-134a | HF-C-134a | HF-C-134a | HF-C-134a | HF-C-134a | HF-C-134a |
| Catalyst | Type | $\alpha$-Al$_2$O$_3$ | $\alpha$-Al$_2$O$_3$ | $\alpha$-Al$_2$O$_3$ | $\alpha$-Al$_2$O$_3$ | $\gamma$-Al$_2$O$_3$ | $\alpha$-Al$_2$O$_3$ | $\alpha$-Al$_2$O$_3$ |
| | Evaluation result of content of $\alpha$-alumina | A | A | A | A | B | A | A |
| | Specific surface area[m$^2$/g] | 3 | 34 | 3 | 3 | 190 | <0.1 | 34 |
| | Pore volume [cm$^3$/g] | 0.01 | 0.16 | 0.01 | 0.01 | 0.48 | <0.01 | 0.16 |
| | Average pore diameter [nm] | 9 | 19 | 18 | 9 | 10 | 0.8 | 19 |
| Contact temperature [°C] | | 450 | 450 | 450 | 450 | 450 | 450 | 450 |
| Pressure [MPaG] | | 0 | 0 | 0 | 0 | 0 | 0 | 0 |
| Molar ratio of Fluorocarbon /N$_2$ [mol/mol] | | 10 | 10 | 10 | 10 | 10 | 10 | 10 |
| Contact time [seconds] | | 4.7 | 4.7 | 4.7 | 4.7 | 4.7 | 4.7 | 4.7 |

(continued)

| | | Example 7 | Example 8 | Example 9 | Example 10 | Example 11 | Example 12 | Example 13 |
|---|---|---|---|---|---|---|---|---|
| Fluorocarbon | | HF-C-134a | HF-C-134a | HF-C-134a | HF-C-134a | HF-C-134a | HF-C-134a | HF-C-134a |
| Contact time [g·sec/mL] | | 19 | 5 | 21 | 11 | 13 | 31 | 5 |
| Conversion Rate [%] | After 10 h | 11.5 | 10.1 | 10.3 | 9.0 | 8.4 | 0.9 | 86.6 |
| | After 50 h | - | - | - | - | 5.8 | - | 69.9 |
| Selectivity [%] | | 98 | 98. | 100 | 97 | 97 | 94 | 87 |

Example 1 to Example 10 and Example 13 are Examples of the present disclosure, and Example 11 to Example 12 are Comparative Examples.

As shown in Table 1 and Table 2, it has been found out, in each of Example 1 to Example 10 and Example 13, that a higher conversion rate is achieved as compared to a conventional method, and that a decrease in the conversion rate is reduced in a production over a long period of time, because the method in each Example includes the step of bringing a fluorocarbon represented by Formula (1) into contact with a catalyst to produce a fluoroolefin represented by Formula (2), and in which method the catalyst contains $\alpha$-alumina, and the catalyst has an average pore diameter of 5 nm or more.

In Example 11, in contrast, a marked decrease in the conversion rate was observed in a production over a long period of time, as compared to Example 1.

Further, in Example 12, the conversion rate was extremely low.

The disclosures of Japanese Patent Application No. 2022-148004 filed on September 16, 2022 and Japanese Patent Application No. 2022-182072 filed on November 14, 2022 are incorporated herein by reference in their entirety. All publications, patent applications, and technical standards mentioned in the present specification are incorporated herein by reference to the same extent as if each individual publication, patent application, or technical standard was specifically and individually indicated to be incorporated by reference.

## Claims

1. A method of producing a fluoroolefin, the method comprising a step of bringing a fluorocarbon represented by the following Formula (1):

$$CX^1X^2F\text{-}CX^3X^4H \qquad (1)$$

into contact with a catalyst to produce a fluoroolefin represented by the following Formula (2):

$$CX^1X^2{=}CX^3X^4 \qquad (2)$$

wherein, in Formula (1) and Formula (2), each of $X^1$, $X^2$, $X^3$ and $X^4$ independently represents a hydrogen atom or a fluorine atom, and at least one of $X^1$, $X^2$, $X^3$ or $X^4$ is a fluorine atom,
wherein the catalyst comprises $\alpha$-alumina, and
wherein the catalyst has an average pore diameter of 5 nm or more.

2. The method of producing a fluoroolefin according to claim 1, wherein the catalyst comprises 65% by mass or more of $\alpha$-alumina.

3. The method of producing a fluoroolefin according to claim 1 or 2, wherein the fluorocarbon is brought into contact with the catalyst at a temperature of from 300°C to 800°C.

4. The method of producing a fluoroolefin according to claim 1 or 2, wherein a contact time between the fluorocarbon and the catalyst is from 0.5 seconds to 100.0 seconds.

5. The method of producing a fluoroolefin according to claim 1 or 2, wherein a contact time between the fluorocarbon and the catalyst is from 1 g·sec/mL to 200 g·sec/mL.

6.  The method of producing a fluoroolefin according to claim 1 or 2, wherein the catalyst has an average pore diameter of from 5 nm to 200 nm.

7.  The method of producing a fluoroolefin according to claim 1 or 2, wherein the catalyst has a pore volume of from 0.002 $cm^3$/g to 1.20 $cm^3$/g.

8.  The method of producing a fluoroolefin according to claim 1 or claim 2, wherein the catalyst is a molded body.

9.  The method of producing a fluoroolefin according to claim 1 or 2, wherein the fluorocarbon is at least one selected from the group consisting of 1,1,1-trifluoroethane, 1,1,2-trifluoroethane, 1,1,2,2-tetrafluoroethane, and 1,1,1,2-tetrafluoroethane.

10. The method of producing a fluoroolefin according to claim 1 or 2, wherein the fluoroolefin is at least one selected from the group consisting of 1,2-difluoroethylene, 1,1-difluoroethylene, and trifluoroethylene.

11. The method of producing a fluoroolefin according to claim 1 or 2, wherein the fluorocarbon is 1,1,1,2-tetrafluoroethane, and the fluoroolefin is trifluoroethylene.

12. The method of producing a fluoroolefin according to claim 1 or 2, wherein:

    the fluorocarbon is brought into contact with the catalyst in the presence of an inert gas, and
    the inert gas is at least one selected from the group consisting of nitrogen, helium, argon, octafluorocyclobutane, and carbon dioxide.

13. The method of producing a fluoroolefin according to claim 1 or 2, further comprising a step of drying the catalyst, before bringing the fluorocarbon into contact with the catalyst.

14. The method of producing a fluoroolefin according to claim 1 or 2, wherein:

    the fluorocarbon is brought into contact with the catalyst in a gas phase, in the presence of water, and
    a concentration of the water is less than 500 ppm with respect to a total amount of a raw material gas containing the fluorocarbon.

15. The method of producing a fluoroolefin according to claim 1 or 2, wherein a conversion rate 10 hours after bringing the fluorocarbon into contact with the catalyst is 30% or less.

# EP 4 588 907 A1

## INTERNATIONAL SEARCH REPORT

International application No.

**PCT/JP2023/023430**

### A. CLASSIFICATION OF SUBJECT MATTER

*C07C 17/25*(2006.01)i; *B01J 21/04*(2006.01)i; *B01J 35/10*(2006.01)i; *C07C 21/18*(2006.01)i; *C07B 61/00*(2006.01)n
FI: C07C17/25; C07C21/18; B01J35/10 301G; B01J21/04 Z; C07B61/00 300

According to International Patent Classification (IPC) or to both national classification and IPC

### B. FIELDS SEARCHED

Minimum documentation searched (classification system followed by classification symbols)

C07C17/25; B01J21/04; B01J35/10; C07C21/18; C07B61/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | CN 103288589 A (TONGJI UNIVERSITY) 11 September 2013 (2013-09-11) claims, paragraphs [0003], [0009]-[0015], examples 1, 2, 4, table 1 | 1-13 |
| Y | | 1-14 |
| A | | 15 |
| X | JP 2004-504283 A (E.I. DU PONT DE NEMOURS AND COMPANY) 12 February 2004 (2004-02-12) claims, paragraphs [0032], [0036]-[0038], [0052], [0054], examples 4, 5, tables 1-19 | 1-13, 15 |
| A | | 14 |
| Y | JP 2019-196347 A (DAIKIN INDUSTRIES, LTD.) 14 November 2019 (2019-11-14) claims, paragraph [0018], example 18, table 3 | 1-14 |
| A | JIA,W. et al. Influence of Lewis Acidity on Catalytic Activity of the Porous Alumina for Dehydrofluorination of 1,1,1,2-Tetrafluoroethane to Trifluoroethylene. Catalysis Letters. 2015, vol. 145, no. 2, pp. 654-661, DOI:10.1007/s10562-014-1409-z entire text, all drawings | 1-15 |

✔ Further documents are listed in the continuation of Box C.     ✔ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **09 August 2023** | **29 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2023/023430**

### C. DOCUMENTS CONSIDERED TO BE RELEVANT

| Category* | Citation of document, with indication, where appropriate, of the relevant passages | Relevant to claim No. |
|---|---|---|
| A | MAO, W. et al. A facile sol-gel synthesis of highly active nano α-aluminum fluoride catalyst for dehydrofluorination of hydrofluorocarbons. Applied Catalysis, B: Environmental. 2017, vol. 206, pp. 65-73, DOI: 10.1016/j.apcatb.2016.12.064<br>entire text, all drawings | 1-15 |
| A | JP 2010-533151 A (SOLVAY FLUOR GMBH) 21 October 2010 (2010-10-21)<br>entire text, all drawings | 1-15 |
| A | WO 2015/147063 A1 (ASAHI GLASS CO LTD) 01 October 2015 (2015-10-01)<br>entire text, all drawings | 1-15 |
| A | JP 2021-183578 A (DAIKIN INDUSTRIES, LTD.) 02 December 2021 (2021-12-02)<br>entire text, all drawings | 1-15 |

Form PCT/ISA/210 (second sheet) (January 2015)

| Patent document cited in search report | | | Publication date (day/month/year) | Patent family member(s) | Publication date (day/month/year) |
|---|---|---|---|---|---|
| CN | 103288589 | A | 11 September 2013 | (Family: none) | |
| JP | 2004-504283 | A | 12 February 2004 | US 2002/0032356 A1 claims, paragraphs [0032], [0036]-[0038], [0052], [0054], examples 4, 5, tables 1-19 CN 1441760 A | |
| JP | 2019-196347 | A | 14 November 2019 | US 2021/0253502 A1 claims, paragraph [0037], example 18, table 3 CN 112088150 A | |
| JP | 2010-533151 | A | 21 October 2010 | US 2010/0191024 A1 entire text, all drawings KR 10-2010-0043234 A CN 101801894 A | |
| WO | 2015/147063 | A1 | 01 October 2015 | US 2017/0008823 A1 entire text, all drawings CN 106164027 A | |
| JP | 2021-183578 | A | 02 December 2021 | US 2023/0101465 A1 entire text, all drawings CN 115667192 A | |

## REFERENCES CITED IN THE DESCRIPTION

**Patent documents cited in the description**

- JP 2019196347 A
- JP 2022148004 A
- JP 2022182072 A

**Non-patent literature cited in the description**

- *Catalysis Letters*, 2015, vol. 145, 654-661